# EUROPÄISCHE PATENTSCHRIFT

(11) **EP 1 229 016 B1**
(45) Veröffentlichungstag und Bekanntmachung des Hinweises auf die Patenterteilung: **11.01.2006**
(21) Anmeldenummer: 01126664.0
(22) Anmeldetag: 08.11.2001
(51) Int. Cl.: C07C 211/63, C08G 18/02, C08G 18/79, C07C 217/58

(54) **Katalysator und Verfahren zur Herstellung von niedrigviskosen und farbreduzierten isocyanatgruppenhaltigen Polyisocyanaten**
Catalyst and process for the preparation of isocyanate groups containing polyisocyanates with low viscosity and reduced colour
Catalyseur et procédé pour la préparation de polyisocyanates à faible viscosité et de coloration réduite

(30) Priorität: 23.12.2000 DE 10065176
(43) Veröffentlichungstag der Anmeldung: 07.08.2002
(73) Patentinhaber: Degussa AG, 40474 Düsseldorf (DE)
(72) Erfinder: Kohlstruk, Stephan, Dr., 48249 Dülmen (DE); Ewald, Michael, 45768 Marl (DE); Bockhoff, Ingo, 45770 Marl (DE); Lomölder, Rainer, Dr., 48153 Münster (DE)

(56) Entgegenhaltungen:
- EP-A- 0 028 696
- EP-A- 0 082 987
- EP-A- 0 351 873
- WO-A-96/10550
- DE-A- 3 902 078
- US-A- 3 980 594
- US-A- 4 040 992
- US-A- 4 748 242
- US-A- 4 960 848
- US-A- 5 240 970
- US-A- 5 288 864
- US-A- 5 691 440
- DATABASE CROSSFIRE BEILSTEIN [Online] Beilstein Institut zur Förderung der Chemischen Wissenschaften, Frankfurt am Main, DE; 1992 Database accession no. 5190380 XP002253686
- DATABASE CROSSFIRE BEILSTEIN [Online] Beilstein Institut zur Förderung der Chemischen Wissenschaften, Frankfurt am Main, DE; Database accession no. 5193770 XP002253687 & TOMAS ET AL.: J. ORG. CHEM., Bd. 61, Nr. 26, 1996, Seiten 9394-9401,
- DATABASE CROSSFIRE BEILSTEIN [Online] Beilstein Institut zur Förderung der Chemischen Wissenschaften, Frankfurt am Main, DE; 1993 Database accession no. 5689067 XP002253688
- DATABASE CROSSFIRE BEILSTEIN [Online] Beilstein Institut zur Förderung der Chemischen Wissenschaften, Frankfurt am Main, DE; 1993 Database accession no. 5701487 XP002253689
- DATABASE CROSSFIRE BEILSTEIN [Online] Beilstein Institut zur Förderung der Chemischen Wissenschaften, Frankfurt am Main, DE; 1993 Database accession no. 6089678 XP002253690
- DATABASE CROSSFIRE BEILSTEIN [Online] Beilstein Institut zur Förderung der Chemischen Wissenschaften, Frankfurt am Main, DE; 1994 Database accession no. 6886506 XP002253731 & SMIRNOVA ET AL.: J. APPL. CHEM. USSR, Bd. 60, Nr. 3, 1987, Seiten 534-539,
- DATABASE CROSSFIRE BEILSTEIN [Online] Beilstein Institut zur Förderung der Chemischen Wissenschaften, Frankfurt am Main, DE; 1997 Database accession no. 7614045 XP002253732 & WANG ET AL.: J. CHEM. KINET, Bd. 28, Nr. 8, 1996, Seiten 615-626,
- PATENT ABSTRACTS OF JAPAN vol. 1997, no. 01, 31. Januar 1997 (1997-01-31) & JP 08 239371 A (MITSUBISHI GAS CHEM CO INC), 17. September 1996 (1996-09-17)

## Beschreibung

Die vorliegende Erfindung betrifft einen Katalysator und ein Verfahren zur Herstellung von niedrigviskosen und farbreduzierten isocyanuratgruppenhaltigen Polyisocyanaten.

Für hochwertige Ein- und Zweikomponenten Polyurethanlacke mit hoher Licht- und Wetterbeständigkeit werden als Isocyanatkomponente insbesondere Isocyanurat- und Uretdiongruppen enthaltende Polyisocyanatmischungen eingesetzt.

Bezüglich der Herstellung von Isocyanurat- und Uretdiongruppen aufweisenden Polyisocyanaten, die als Rohstoffe für Polyurethan-Lackformulierungen in Frage kommen, sind unterschiedliche Verfahren bekannt. Diese Verfahren unterscheiden sich in der Regel durch die Auswahl der Trimersierungskatalysatoren oder auch durch die Auswahl der bei der Oligomerisierungsreaktion einzusetzenden organischen Isocyanate (vgl. z.B. GB-PS 1391066, EP 82 987, DE 390 2078, EP 339 396, EP 224 165).

Zur Trimerisierung geeignete Isocyanate, z. B. aromatische, cycloaliphatische und aliphatische di- und höherwertige Polyisocyanate, können nach verschiedenartigen Verfahren hergestellt werden (Annalen der Chemie 562 (1949), Seiten 75ff). Technisch insbesondere bewährt hat sich die Herstellung durch Phosgenierung von organischen Polyaminen zu den entsprechenden Polycarbaminsäurechloriden und deren thermische Spaltung in organische Polyisocyanate und Chlorwasserstoff. Alternativ können organische Polyisocyanate auch ohne die Verwendung von Phosgen, d. h. nach phosgenfreien Verfahren, hergestellt werden. Nach Angaben der EP-B-0 126 299 (US-A-4 596 678), EP-B-0 126 300 (US-A-4 596 679) und EP-A-0 355 443 (US-A-5 087 739) beispielsweise können (cyclo)aliphatische Diisocyanate - wie 1,6-Hexamethylendiisocyanat (HDI) und/oder isomere aliphatische Diisocyanate mit 6 Kohlenstoffatomen im Alkylenrest und 1-Isocyanato-3-isocyanato-methyl-3,5,5-trimethyl-cyclohexan (Isophorondiisocyanat bzw. IPDI) - hergestellt werden durch Umsetzung der zugrundeliegenden (cyclo)aliphatischen Diamine mit Harnstoff und Alkoholen zu (cyclo)aliphatischen Biscarbaminsäureestem und deren thermische Spaltung in die entsprechenden Diisocyanate und Alkohole. Die Synthese erfolgt kontinuierlich in einem Kreislaufverfahren und gegebenenfalls in Gegenwart von N-unsubstituierten Carbaminsäureestem, Dialkylcarbonaten und anderen aus dem Reaktionsprozeß zurückgeführten Nebenprodukten.

Als Katalysatoren für die Trimerisierung von Isocyanaten zu den angestrebten Isocyanurat- und Uretdiongruppen aufweisenden Polyisocyanaten können beispielsweise tertiäre Amine, Phosphine, Alkaliphenolate, Aminosilane, quartäre Ammoniumhydroxide oder quartäre Ammoniumcarbonate eingesetzt werden. Gut geeignete Oligomerisierungskatalysatoren sind auch Hydroxide, Halogenide oder Carboxylate von Hydroxyalkylammonium-Ionen (vgl. z.B. EP 351 873, US 5 290 902), Alkalimetallsalze sowie Zinn-, Zink- bzw. Bleisalze von Alkylcarbonsäuren. In Abhängigkeit vom Katalysator ist auch die Verwendung von diversen Co-Katalysatoren wie z.B. OH-funktionalisierten Verbindungen oder Mannichbasen aus sekundären Aminen und Aldehyden bzw. Ketonen möglich.

Zur Oligomerisierung läßt man die (cyclo)aliphatischen Diisocyanate in Gegenwart des Katalysators, gegebenenfalls unter Verwendung von Lösungsmitteln und/oder Hilfsstoffen, bis zum Erreichen des gewünschten Umsatzes reagieren. Man spricht in diesem Zusammenhang auch von partieller Trimerisierung, da der angestrebte Umsatz in der Regel deutlich unterhalb von 100% liegt. Danach wird die Reaktion durch Desaktivierung des Katalysators abgebrochen und das überschüssige monomere Diisocyanat üblicherweise abgetrennt, in der Regel durch Kurzweg- oder Dünnschichtdestillation. Die Desaktivierung erfolgt thermisch oder durch Zusatz eines Katalysatorinhibitors wie beispielsweise p-Toluolsulfonsäure oder Bis(2-Ethylhexyl)phosphat. Besonders vorteilhaft im Hinblick auf die Trimerisierung von Isocyanaten im technischen Maßstab ist der Einsatz von quartämeren Hydroxyalkylammoniumcarboxylaten als Oligomerisierungskatalysatoren. Diese Katalysatoren vom Cholin-Typ sind thermisch labil. Es ist unnötig, die Trimerisierung bei Erreichen des gewünschten Umsatzes durch Zusatz potentiell qualitätsmindernder Katalysatorinhibitoren abzustoppen. Statt dessen erlaubt die gezielte thermische Deaktivierung eine optimale Prozeßkontrolle. Vorteilhaft ist die Thermolabilität auch unter dem Gesichtspunkt der Prozeßsicherheit. Ein unkontrolliertes "Durchgehen" der Reaktion ist ausgeschlossen, sofern die zudosierte Katalysatormenge das übliche Maß nicht um ein Vielfaches übersteigt.

Je nach verwendetem Katalysatortyp und Reaktionstemperatur erhält man Polyisocyanate mit unterschiedlichen Anteilen an Isocyanurat- bzw. Uretdiongruppen. Die Produkte sind meist klare, aber in Abhängigkeit vom Katalysatortyp, der Diisocyanatqualität, der Reaktionstemperatur und der Reaktionsfahrweise mehr oder weniger stark gelbgefärbte Produkte. Für die Herstellung hochwertiger Polyurethanlacke sind jedoch Produkte mit einer möglichst niedrigen Farbzahl erwünscht.

Angesichts anhaltender legislativer Bemühungen zur Kontrolle und Beschränkung der Emission von flüchtigen organischen Verbindungen (sogenannte "volatile organic compounds" bzw. "VOCs") stehen die Lackhersteller kontinuierlich unter dem Druck, den Lösemittelgehalt ihrer Lackformulierungen zu reduzieren. Die Einhaltung der strengen gesetzlichen Forderungen ist keine triviale Aufgabe. Lackformulierungen werden mit Hilfe des Lösemittels auf eine Viskosität eingestellt, die optimale Verarbeitbarkeit und Sprühfähigkeit gewährleistet. Wird der Lösemittelgehalt reduziert, steigt die Viskosität zwangsläufig an und die Verarbeitungsparameter der Formulierung werden signifikant beeinträchtigt. Dem Problem läßt sich jedoch begegnen, wenn zur Herstellung der lösemittelarmen Systeme, sogenannter "Low-VOC"-Lacke, besonders niedrigviskose Bindemittelkomponenten eingesetzt werden. Seitens der Hersteller von PUR-Formulierungen besteht demnach dringender Bedarf an isocyanuratgruppenhaltigen Polyisocyanaten guter Farbqualität, die sich zudem durch eine geringe Viskosität auszeichnen. Letzteres gilt insbesondere für Polyisocyanate auf Basis von IPDI (Isophorondiisocyanat) und NBDI (2,5(2,6)-Bis(isocyanatomethyl)-bicyclo[2.2.1]heptan), die in monomerbefreiter Form eine besonders hohe Viskosität aufweisen.

Bei der Trimerisierung von Diisocyanaten entstehen neben dem idealen Trimer (Monoisocyanurat) auch das Pentamer, das Heptamer und höhere Oligomere. Dabei nimmt die Viskosität des entmonomerisierten Polyisocyanats mit steigenden Oligomergehalt zu. Der Oligomergehalt eines isocyanuratgruppen enthaltenden Polyisocyanats verhält sich grundsätzlich umgekehrt proportional zum Umsatz; er läßt sich also über den Umsatz der partiellen Trimeriserung kontollieren. Wird ein geeignet niedriger Umsatz angesteuert, wird auch ein niedrigviskoses Produkt erhalten. Diese Vorgehensweise ist jedoch sehr unwirtschaftlich.

US Pat. Nr. 5,691,440 beschreibt Trimerisierungskatalysatoren, die unabhängig vom Verfahren bei vergleichbarem Umsatz einen geringeren Oligomergehalt und damit ein niedrigviskoseres Produkt liefern, als die Katalysatoren des Stands der Technik (S.2, Z.67 - S.3, Z.3). Mit Hilfe dieser Katalysatoren, bei denen es sich um eine begrenzte Auswahl spezieller Tetraalkylammoniumcarboxylate handelt (Anspruch 1), läßt sich selbst das anspruchsvolle IPDI auch noch bei hohen und damit wirtschaftlichen Umsätzen zu einem oligomerarmen Produkt trimerisieren (S.3, Z.3 - S.3, Z.37).

Die Katalysatoren des US-Pat. 5,691,440 ermöglichen somit den wirtschaftlichen Zugang zu niedrigviskosen isocyanuratgruppenhaltigen Polyisocyanaten, selbst wenn anspruchsvolle Diisocyanate wie IPDI als Rohstoff zum Einsatz gelangen. Nachteilig ist jedoch, daß die Produkte eine unerwünschte Gelbfärbung aufweisen. Ihre Farbqualität ist optimierungsbedürftig, da - wie bereits erwähnt - für die Herstellung hochwertiger Polyurethanlacke Produkte mit einer möglichst niedrigen Farbzahl erwünscht sind.

US-4,960,848 betrifft ein Verfahren zur Herstellung von isocyanatgruppenhaltigen Polyisocyanaten. Dieses Verfahren unterscheidet sich von der vorliegenden Anmeldung darin, dass Ammoniumfluoride als Katalysator verwendet werden.

US-5,691,440 betrifft ein Verfahren zur Herstellung von isocyanatgruppenhaltigen Polyisocyanaten. Obwohl die Ammoniumcarboxylate der vorliegenden Anmeldung unter Formel I von US 5,691,440 fallen, sind diese nicht explizit in US 5,690,848 erwähnt.

US-3,980,594 betrifft ein Verfahren zur Herstellung von isocyanatgruppenhaltigen Polyisocyanaten unter Verwendung von Bis(trimethylbenzylammonium)maleat (Spalte 3, Zeilen 44 und 45).

Überraschenderweise wurde nun gefunden, daß spezielle quartärnere Benzylammoniumcarboxylate in der Lage sind, niedrigviskose isocyanuratgruppenhaltige Polyisocyanate mit deutlich verbesserter Farbgüte zu liefern.

Gegenstand der Erfindung ist ein Verfahren zur Herstellung von niedrigviskosen und farbreduzierten isocyanuratgruppenhaltigen Polyisocyanaten durch partielle Trimerisierung von aliphatischen und/oder cycloaliphatischen Diisocyanaten und anschließender Abtrennung von überschüssigem Düsocynat, dadurch gekennzeichnet, dass die Trimerisierung in Gegenwart von 0.02 - 2 Gew.-%, basierend auf dem Gewicht des eingesetzten Diisocyanates, mindestens eines Trimerisierungskatalysators der allgemeinen Formel (I) erfolgt 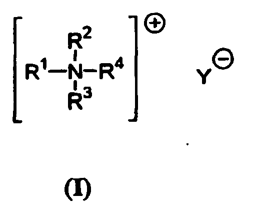
mit
R¹= 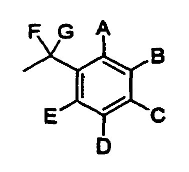
und wobei die Variablen folgende Bedeutung haben:
A,B,C,D,E sind gleichzeitig oder unabhängig voneinander Wasserstoff, Chlor, Cl-C4-Alkyl, C1-C4-Alkoxy, C1-C4-Alkoxycarbonyl, Hydroxy, (R⁵)₃SiO-, (R⁵)₂N-, -COOH, (R⁵)₂N-CH₂- oder Phenyl, wobei zwei beliebige benachbarte Reste aus der Gruppe A,B,C,D,E einen gemeinsamen 5- oder 6-gliedrigen, gesättigten oder ungesättigten Ring bilden können, der auch Stickstoff-, Schwefel- oder Sauerstoff-Heteroatome enthalten kann;
   F steht für Wasserstoff oder Methyl oder Fluor;
   G steht für Wasserstoff oder Methyl oder Fluor;
   R² und R³ sind gleichzeitig oder unabhängig voneinander C1-C18-Alkyl oder R¹;
   R⁴ ist Wasserstoff, Methyl, C2-C18-Alkyl, C3-C8-Cycloalkyl oder C2-C12-Alkoxy;
   R⁵ ist C1-C18-Alkyl;
   Y⁻ steht für R⁶COO⁻;
   R⁶ steht für Wasserstoff oder einen verzweigten oder unverzweigten aliphatischen oder araliphatischen C1-C12-Alkylrest.

Die Herstellung der niedrigviskosen Isocyanuratgruppen enthaltenden Polyisocyanate durch partielle Trimerisierung kann kontinuierlich (Rohrreaktor oder Kesselkaskade) erfolgen oder auch batchweise durchgeführt werden. Die erfindungsgemäßen Katalysatoren werden in geringer Konzentration zwischen 0.02 und 2.0 Gew.-%, eingesetzt. Die exakte Menge ist abhängig vom individuellen Katalysator, vom Umsatzziel und von der Verfahrensweise. Die Deaktivierung des Katalysators kann thermisch oder chemisch erfolgen. Zur chemischen Inhibierung geeignet sind beispielsweise HCl, Benzoylchlorid oder Dibutylphosphat. Die Deaktivierung des Katalysators, ob thermisch oder chemisch, ist notwendig, um die Trimerisierung abzubrechen und die Lagerstabilität des Polyisocyanats zu gewährleisten.

Die Trimerisierung läßt sich unter diesen Bedingungen innerhalb von 1-40 Minuten durchführen. Es werden Verbindungen erhalten, die einen oder auch mehrere Isocyanuratringe haben. Als Nebenkomponenten können in geringer Menge ggf. auch Verbindungen mit Uretdionstruktur gefunden werden. Verbindungen dieser Art sind in der Literatur beschrieben.

Als zur Trimerisierung geeignete Ausgangsverbindungen kommen Diisocyanate mit aliphatischen, cycloaliphatischen oder aliphatische und cycloaliphatischen Isocyanatgruppen in Frage, die nach dem Phosgenverfahren oder einem phosgenfreien Prozeß hergestellt worden sind, oder auch Mischungen solcher Diisocyanate. Geeignete aliphatische Diisocyanate weisen bevorzugt 3 bis 16, besonders bevorzugt 4 bis 12 Kohlenstoffatome, in ihrer linearen oder verzweigten Alkylen-Substruktur auf Geeignete cycloaliphatische Diisocyanate haben bevorzugt 4 bis 18, besonders bevorzugt 6 bis 15 Kohlenstoffatome, in ihrer Cycloalkylen-Substruktur. Geeignete Ausgangsdiisocyanate sind aliphatische und/oder cycloaliphatische Diisocyanate, z.B. 1,4-Diisocyanatocyclohexan, 1,6-Diisocyanatohexan (HDI), 1,12-Diisocyanatododecan, 1-Isocyanato-3,3,5-trimethylcyclohexan (IPDI), 4,4'-Diisocyanatodicyclohexylmethan, 1,5-Diisocyanato-2,2-dimethylpentan, 1,5-Diisocyanato-2-ethyl-2-propylpentan, 1,5-Diisocyanato-2-butyl-2-ethylpentan 1,6-Diisocyanato-2,4,4-trimethylhexan und 1,6-Diisocyanato-2,4,4-trimethylhexan (TMDI), 1,5-Diisocyanato-2-methylpentan (MPDI) sowie 2,5(2,6)-Bis(isocyanatomethyl)bicyclo[2.2.1]heptan (NBDI). Bevorzugt sind 1-Isocyanato-3,3,5-trimethyl-5-isocyanatomethylcyclohexan (IPDI), 1,6-Hexamethylendiisocyanat (HDI), 2-Methyl-1,5-diisocyanat (MPDI), 2,5(2,6)-Bis(isocyanatomethyl)bicyclo[2.2.1]heptan (NBDI) sowie 2,2,4- oder 2,4,4-Trimethyl-1,6-hexamethylendiisocyanat (TMDI).

Zur Herstellung isocyanuratgruppenhaltiger Polyisocyanate werden die erfindungsgemäßen Katalysatoren bevorzugt in kleinen Mengen eingesetzt. Die exakte Menge läßt sich leicht experimentell ermitteln und ist abhängig von der katalytischen Aktivität des individuellen Kontaktes, dem Umsatzziel und der Verfahrensweise.

Erfindungsgemäß werden die quart. Benzylammoniumcarboxylate der Formel I im allgemeinen in einer Menge von 0.02 - 2 Gew.-%, bevorzugt 0.04 - 1.5 Gew.-% , basierend auf dem Gewicht des eingesetzten (cyclo)aliphatischen Düsocyanates, eingesetzt.

Die Trimerisierung der Diisocyanate wird entweder batchweise oder kontinuierlich durchgeführt.

Beim Batchverfahren wird in einem Rührreaktor gearbeitet. Dabei wird die Mischung aus Diisocyanat und Katalysator üblicherweise bei Raumtemperatur vorgelegt. Im Anschluß wird die Temperatur der Reaktionsmischung zur Initiierung der Trimerisierung auf 35-140°C, bevorzugt auf 50-110°C, erhöht. Alternativ kann der Katalysator auch zudosiert werden, nachdem das Diisocyanat die zur Reaktion notwendige Temperatur erreicht hat. Die Trimerisierung ist exotherm, der Katalysator wird im Verlauf der Reaktion zerstört.

Die kontinuierliche Trimerisierung wird zweckmäßigerweise in einer Reaktionsschlange unter kontinuierlicher, gleichzeitiger Zudosierung des Diisocyanats und des Trimerisierungskatalysators bei 40-120°C und innerhalb von 1-40 Minuten durchgeführt. Es hat sich als besonders zweckmäßig erwiesen, die Einsatzprodukte vor Eintritt in die Reaktionsschlange intensiv zu mischen. Zur exakteren Dosierung der geringen Katalysatormengen und zur Erzeugung einer besseren Durchmischungsqualität kann es vorteilhaft sein, den Katalysator in einem geeigneten organischen Lösungsmittel zu lösen. Geeignet sind prinzipiell solche Lösungsmittel, in denen der Katalysator eine gute Löslichkeit hat. Vorzugsweise wird jedoch weitgehend auf den Einsatz von Lösungsmitteln verzichtet.

Die kontinuierlich Trimerisierung kann auch in einer Rührkesselkaskade durchgeführt werden. In diesem Fall ist es vorteilhaft, die Trimeriserung isotherm bei einer Temperatur zwischen 40°C und 110°C, bevorzugt 60°C und 90°C, durchzuführen und die Umsetzung bei Erreichen des gewünschten Umsatzes durch Zudosierung eines Katalysatorinhibitors abzustoppen. Auch eine Kopplung von Rührkesselkaskade und Reaktionsschlange ist denkbar.

Zur Entfernung des nicht umgesetzten Diisocyanats wird das Reaktionsgemisch einer Kurzwegverdampfung unterzogen.

Die erfindungsgemäß hergestellten monomerbefreiten isocyanuratgruppenhaltigen Isocyanurate stellen nützliche Zwischenprodukte für Polyurethanbeschichtungen, z.B. Leder- und Textilbeschichtungen, und für Polyurethandispersionen und Klebstoffe dar, und sind besonders wertvoll als Polyisocyanat-Komponente in 1- und 2-Komponenten Polyurethansystemen für wetter- und lichtbeständige Polyurethanlacke. Dabei können die erfindungsgemäßen Verfahrenprodukte sowohl als solche als auch in mit Blockierungsmitteln blockierter Form zum Einsatz gelangen. Geeignete Blockierungsmittel sind dabei beispielsweise Lactame wie ε-Caprolactam, Oxime wie Methylethylketoxim oder Butanonoxim, Triazole wie 1H-1,2,4-Triazol, leicht enolisierbare Verbindungen wie Acetessigester oder Acetylaceton oder auch Malonsäurederivate wie Malonsäurediester mit 1-10 C-Atomen in den Alkoholresten.

Gegenstand der Erfindung sind auch Verbindungen, verwendbar als Katalysatoren zur Trimerisierung von Diisocyanaten der allgemeinen Formel (I) 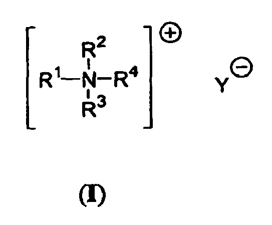
mit
R¹= 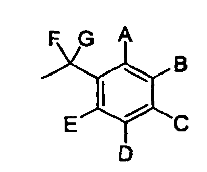
und wobei die Variablen folgende Bedeutung haben:
A: Methyl, Methoxy, H;
B: H;
C: Methyl, Methoxy, H;
D: H,
E: Methyl, Methoxy, H;
F: H, Methyl;
G: H, Methyl;
R², R³: R¹ oder Methyl, Ethyl, Propyl, Butyl, Pentyl, Hexyl;
R⁴, R⁵: Methyl, Ethyl, Propyl, Butyl, Pentyl, Hexyl, Cyclohexyl;
Y⁻ steht für R⁶COO⁻ ;
R⁶: tert. Butyl, Pentyl, iso-Pentyl, Hexyl, iso-Hexyl, Ethylpentyl, iso-Heptyl.

Ebenfalls Gegenstand der Erfindung ist ein Verfahren zur Herstellung der Katalysatoren, wobei in einem ersten Schritt ein tertiäres Amin quaternisiert wird und in einem zweiten Schritt die erhaltene quaternäre Benzylammoniumverbindung in das entsprechende Carboxylat überführt wird.

Die erfindungsgemäßen Trimerisierungskatalysatoren lassen sich zur Umsetzung von Diisocyanaten einsetzen, die nach dem Phosgenverfahren oder einem phosgenfreien Prozeß, zum Beispiel durch thermische Spaltung von (cyclo)aliphatischen Biscaraminsäureestern (vgl. z.B. EP-B-0 126 299 (US-A-4 596 678)), hergestellt worden sind. Geeignete Ausgangsdiisocyanate sind aliphatische und/oder cycloaliphatische Diisocyanate, z.B. 1,4-Diisocyanatocyclohexan, 1,6-Diisocyanatohexan (HDI), 1,12-Diisocyanatododecan, 1-Isocyanato-3,3,5-trimethylcyclohexan (IPDI), 4,4'-Diisocyanatodicyclohexylmethan, 1,5-Diisocyanato-2,2-dimethylpentan, 1,5-Diisocyanato-2-ethyl-2-propylpentan, 1,5-Diisocyanato-2-butyl-2-ethylpentan 1,6-Diisocyanato-2,4,4-trimethylhexan und 1,6-Diisocyanato-2,4,4-trimethylhexan (TMDI), 1,5-Diisocyanato-2-methylpentan (MPDI) sowie 2,5(2,6)-Bis(isocyanatomethyl)bicyclo[2.2.1]heptan (NBDI).

Zur Herstellung der erfindungsgemäßen Trimerisierungskatalysatoren kann ein zweistufiger Syntheseweg beschritten werden. Im ersten Schritt wird ein tertiäres Amin quarternisiert. Als Alkylierungsmittel bieten sich Alkylhalogenide, Benzylhalogenide, Alkyltriflate, Benzyltriflate und auch Alkyl- und Benzyltosylate an. Geeignet sind z. B. Methlyiodid, Methylbromid, Methlychlorid, Methyltriflat, Methyltosylat, Ethyliodid, Ethylbromid, Ethylchlorid, Ethyltriflat, Ethyltosylat, Butyliodid, Butylbromid, Butylchlorid, Butyltriflat, Butyltosylat, Dodecyliodid, Dodecylbromid, Dodecylchlorid, Dodecyltriflat, Dodecyltosylat, Allyliodid, Allylbromid, Allylchlorid, Allyltriflat, Allyltosylat, Benzyliodid, Benzylbromid, Benzylchlorid, Benzyltriflat, Benzyltosylat, 4-Chlorbenzyliodid, 4-Chlorbenzylbromid, 4-Chlorbenzylchlorid, 4-Chlorbenzyltriflat, 4-Chlorbenzyltosylat, 2-Chlorbenzyliodid, 2-Chlorbenzylbromid, 2-Chlorbenzylchlorid, 2-Chlorbenzyltriflat, 2-Chlorbenzyltosylat, Methallyliodid, Methallylbromid, Methallylchlorid, Methallyltriflat, Methallyltosylat, 4-Methoxycarbonylbenzyliodid, 4-Methoxycarbonylbenzylbromid, 4-Methoxycarbonylbenzylchlorid, 4-Methoxycarbonylbenzyltriflat, 4-Methoxycarbonylbenzyltosylat, 4-Methoxybenzyliodid, 4-Methoxybenzylbromid, 4-Methoxybenzylchlorid, 4-Methoxybenzyltriflat, 4-Methoxybenzyltosylat, 4-Methylbenzyliodid, 4-Methylbenzylbromid, 4-Methylbenzylchlorid, 4-Methylbenzyltriflat, 4-Methlybenzyltosylat, 3-Methylbenzyliodid, 3-Methylbenzylbromid, 3-Methylbenzylchlorid, 3-Methylbenzyltriflat, 3-Methlybenzyltosylat, 2-Methylbenzyliodid, 2-Methylbenzylbromid, 2-Methylbenzylchlorid, 2-Methylbenzyltriflat und 2-Methylbenzyltosylat. Geeignete Alkylierungsmittel sind auch Meerweinsalze wie Trimethyloxoniumtetrafluoroborat und Triethlyoxoniumtetrafluoroborat. Beispiele für grundsätzlich geeignete tertiäre Amine sind Trimethylamin, Triethylamin, Dimethylethylamin, Diethylmethylamin, Tripropylamin, Tributylamin, Trioctylamin, Tridodecylamin, N,N-Dimethylallylamin, N,N-Diethylallyamin, N-Ethyl-N-Methylallylamin, N,N-Dimethylmethallyamin, N,N-Diethylmethallyamin, N-Ethyl-N-Methymethallyamin, Tridodecylamin, Dimethyldodecylamin, Diethyldodecylamin, Dipropyldodecylamin, Dibutyldodecylamin, Didodecylmethylamin, Didodecylethylamin, Didodecylpropylamin, Didodecylbutylamin, N,N-Dimethyl-2-methoxybenzylamin, N,N-Dimethyl-3-methoxybenzylamin, N,N-Dimethyl-4-methoxybenzylamin, N,N-Dimethyl-2,3-dimethoxybenzylamin, N,N-Dimethyl-3,4-dimethoxybenzylamin, N,N-Dimethyl-3,5-dimethoxybenzylamin, N,N-Dimethylbenzylamin, N,N-Dimethylbenzylamin-4-carbonsäurenitril, 4-Methoxycarbonyl-N,N-Dimethylbenzylamin, 4-Ethoxycarbonyl-N,N-Dimethylbenzylamin, 3-(N,N-Dimethylaminomethyl)-N,N-dimethylbenzylamin, 1-Phenylethyldimethylamin, 4-Hydroxy-N,N-dimethylbenzylamin, 4-Trimethylsiloxy-N,N-dimethylbenzylamin und N,N-Dimethylnaphtylamin. Die Quarternisierung der tert. Amine erfolgt bei 0°C bis 100°C und kann in Gegenwart oder Abwesenheit von Lösemitteln durchgeführt werden. Der Lösemittelbasierende Prozess wird in der Regel bevorzugt. Geeignete Lösemittel sind z.B. Acetonitil, Dimethylformamid, Dimethylsulfoxid, Aceton, 2-Ethylhexandiol und Dichlormethan.
Die erhaltenen quart. Benzylammoniumhalogenide, -tosylate, -triflate oder -tetrafluoroborate werden im zweiten Schritt in die angestrebten quart. Benzylammoniumcarboxylate überführt. Dies geschieht vorzugsweise mit Hilfe der Ionenaustauschchromatographie. Dazu wird ein basisches Ionenaustauscherharz (z.B. Amberlyst-, Dowex- oder Sephadex-Typ) mit Kalilauge oder Natronlauge aktiviert und mit der gewünschten Carbonsäure beladen. Als geeignete Carbonsäuren kommen beispielsweise Pivalinsäure, Hexansäure, Essigsäure, 2-Ethylhexansäure, Propansäure, Adipinsäure, Bernsteinsäure und Ölsäure in Frage. Danach wird die Chromatographiesäule mit dem quart. Benzylammoniumsalz beschickt und eluiert. Das Eluat enthält das erfindungsgemäße quart. Benzylammoniumcarboxylat. Das Lösemittel kann durch Anlegen von Vakuum entfernt werden. Im Falle der quart. Benzylammoniumhalogenide lassen sich die erfindungsgemäßen Katalysatoren auch durch Kationenaustausch in Lösung in sehr reiner Form erhalten, wenn als Reaktionspartner die den aufgeführten Carbonsäuren zugrundeliegenden Silbercarboxylate eingesetzt werden. Es ist auch möglich, die quart. Benzylammoniumhalogenide, -tosylate, -triflate oder -tetraborate mit Hilfe der Ionenaustauschchromoatographie zunächst in die entsprechenden quart. Benzylammoniumhydroxide zu konvertieren und diese im Anschluß durch Umsetzung mit der gewünschten Carbonsäure und ggf. Entfernung des freigesetzten Wassers in die erfindungsgemäßen quart. Benzylammoniumcarboxylate zu überführen.

### Beispiele

### A. Herstellung der Katalysatoren

Alle Umsetzungen erfolgten unter einer Inertgas-Atmosphäre, vorzugsweise unter Stickstoff.

### A.1. Herstellung von Benzyldimethylethylammoniumpivalat (Kat.1)

In einem Dreihalskolben mit Claisen-Aufsatz, Rückflußkühler, Innenthermometer, mechanischem Rühraufsatz, Tropftrichter sowie Gasein- und -auslaß wurde Benzyldimethylamin (0,2 mol) bei Raumtemperatur unter Rühren tropfenweise so mit Triethyloxoniumtetrafluoroborat (200 ml einer 1M-Lösung in Methylenchlorid)), daß die Temperatur der Reaktionsmischung 45°C nicht überstieg. Nach 7d wurde das Methlyenchlorid im Vakuum abgezogen und der viskose, hellgelbe Rückstand in Methanol aufgenommen.

Eine Chromatographiesäule (Durchmesser ca. 3,5 cm) wurde mit Dowex 1X8-50 befiillt und nacheinander mit einer wäßr. 2M NaOH-Lösung, einer 3%igen Lösung von Pivalinsäure in Ethanol, Methanol und der methanolischen Lösung des quart. Ammoniumtetrafluoroborats beschickt. Der erfindungsgemäße Katalysator wurde mit MeOH eluiert, das Eluat im Vakuum eingeengt. Der Rückstand wurde in Diethylether aufgenommen, der unlösliche Bestandteil (ca. 2,8 g) abfiltriert und der Ether im Vakuum entfernt. Ausbeute: 38,2 g (72%) Kat.1 als hellgelbe, viskose Flüssigkeit.

### A.2. Herstellung von Benzyldimethylethylammonium-2-ethylhexanoat (Kat.2)

Die Herstellung erfolgte analog zu Kat.1 (A.1.). Anstelle von Pivalinsäure wurde 2-Ethylhexansäure eingesetzt. Ausbeute: 33,5 g (55%) Kat.2 als hellgelbe, viskose Flüssigkeit.

### A.3. Herstellung von Benzyltributylammonium-2-ethylhexanoat (Kat.3)

In einem Dreihalskolben mit Claisen-Aufsatz, Innenthermomether, Tropftrichter und mechanischem Rührer sowie Gasein- und -auslaß wurde eine Lösung von Tributylamin (0,2 mol) in 70 ml Dimethylformamid unter Rühren tropfenweise mit Benzylchlorid versetzt, so daß die Temperatur der Reaktionsmischung 50°C nicht überstieg. Nach vollständiger Zudosierung wurde die Reaktionstemperatur auf 60-70°C erhöht. Nach 6 h wurde das Lösemittel im Vakuum entfernt und der Rückstand in Wasser aufgenommen. Der wäßr. Lösung des quart. Ammoniumchlorids wurde unter Rühren tropfenweise Silber-2-ethylhexanoat (0,2 mol; aus 2-Ethylhexansäure und Sibercarbonat) zugesetzt. Man ließ über Nacht stehen und filtrierte den Niederschlag ab. Das Filtrat wurde im Vakuum bis zur Trockne eingeengt. Ausbeute: 82,1 g (98%) Kat.3 als hellgelbes, viskoses Öl.

### A.4. Herstellung von N,N-Dimethyl-N-ethyl-N-(4-methoxybenzyl)ammonium-2-ethylhexanoat (Kat.4)

Die Herstellung erfolgte analog zu Kat. (A.1.). Anstelle von Pivalinsäure wurde 2-Ethylhexansäure, anstelle von Benzyldimethylamin wurde N,N-Dimethyl-N-(4-methoxybenzyl)amin eingesetzt. Ausbeute: 45,3 g (67%) Kat.4 als hellgelbes, viskoses Öl.

### A.5. Herstellung von N,N,N-Tributyl-N-(4-methoxybenzyl)ammoniumpivalat (Kat.5)

Die Herstellung erfolgte analog zu Kat.3 (A.3.). Anstelle von 2-Ethylhexansäure wurde Pivalinsäure, anstelle von Benzylchlorid wurde 4-Methoxybenzylchlorid eingesetzt. Ausbeute: 79,0 g (97%) Kat.5 als hellgelbes, viskoses Öl.

### B. Trimerisierungsbeispiele: Beispiele 1-10 und Vergleichsbeispiele A-E

### B.1. Trimerisierung der Isocyanate

Katalysator und (cyclo)aliphatisches Diisocyanat werden bei Raumtemperatur vorgelegt. Die Temperatur der unter Inertgas-Atmosphäre (N₂) gehaltenen, mechanisch gerührten Reaktionsmischung wird innerhalb von 10-15 min kontinuierlich auf die Starttemperatur (ca. 70°C) erhöht. Nach Initiierung der exothermen Trimerisierungsreaktion wird die Heizquelle entfernt. Die Temperatur der Reaktionsmischung durchläuft ein Maximum und fällt nach der im Verlauf der Umsetzung erfolgenden thermischen Deaktivierung des Katalysators wieder ab. Man läßt auf Raumtemperatur abkühlen und trennt das überschüssige Monomer vom Polyisocyanat durch Kurzwegverdampfung ab.

Als Vergleichskatalysatoren wurden solche eingesetzt, die im US-Pat. 5,691,440 beschrieben werden: N-(2-Hydroxypropyl)-N,N,N-trimethylammonium-2-ethylhexanoat (V-Kat.1), Aliquat 336-pivalat (V-Kat.2) und Tetrabutylammoniumpivalat (V-Kat.3). Die Katalysatoren wurden nach den Vorgaben des US-Pat. 5,691,440 synthetisiert.

Die Ergebnisse der Trimerisierungsversuche sind in Tabelle 1. zusammengefaßt und belegen das Leistungsvermögen der erfindungsgemäßen Katalysatoren in bezug auf Viskosität und Farbe der gebildeten Polyisocyanate. Alkohole wie Benzylalkohol oder Methanol können als Co-Katalysatoren oder zur Verdünnung der erfindungsgemäßen Katalysatoren eingesetzt werden.

### Tabelle 1.

| Eintrag | Katalysator | Umsatz [%] | Viskosität bei 23°C¹⁾ [mPas] | Farbzahl²⁾ [Hazen] |
|---|---|---|---|---|
| Vergleichsbeispiel | | | | |
| A | V-Kat.1 | 35 | 914 | 110 |
| B | V-Kat.1 | 44 | 983 | 102 |
| C | V-Kat.2 | 34 | 523 | 83 |
| D | V-Kat.2 | 43 | 732 | 98 |
| E | V-Kat.3 | 40 | 556 | 83 |

| Beispiel | | | | |
|---|---|---|---|---|
| 1 | Kat.1 | 33 | 503 | 51 |
| 2 | Kat.1 | 48 | 697 | 39 |
| 3 | Kat.2 | 35 | 529 | 56 |
| 4 | Kat.2 | 50 | 709 | 47 |
| 5 | Kat.3 | 25 | 604 | 77 |
| 6 | Kat.3 | 34 | 740 | 73 |
| 7 | Kat.4 | 36 | 525 | 60 |
| 8 | Kat.4 | 43 | 632 | 47 |
| 9 | Kat.5 | 35 | 532 | 46 |
| 10 | Kat.5 | 40 | 609 | 38 |

| | | | | |
|---|---|---|---|---|
| ¹⁾monomerbefreites Polyisocyanat auf Basis von Isophorondiisocyanat, 70%ig in Butylacetat; | | | | |
| ²⁾monomerbefreites Polyisocyanat auf Basis von Isophorondiisocyanat, 50%ig in Butylacetat | | | | |

## Patentansprüche

1. Verfahren zur Herstellung von niedrigviskosen und farbreduzierten isocyanuratgruppenhaltigen Polyisocyanaten durch partielle Trimerisierung von aliphatischen und/oder cycloaliphatischen Diisocyanaten und anschließender Abtrennung von überschüssigem Diisocynat,
**dadurch gekennzeichnet,**
**dass** die Trimerisierung in Gegenwart von 0.02 - 2 Gew.-%, basierend auf dem Gewicht des eingesetzten Diisocyanates, mindestens eines Trimerisierungskatalysators der allgemeinen Formel (**I**) erfolgt 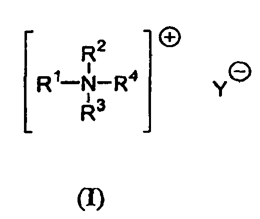
mit
R¹= 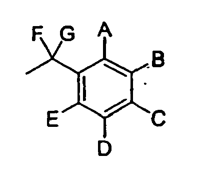
und wobei die Variablen folgende Bedeutung haben:
A: Methyl, Methoxy, H;
B: H;
C: Methyl, Methoxy, H;
D: H,
E: Methyl, Methoxy, H;
F: H, Methyl;
G: H, Methyl;
R², R³: R¹ oder Methyl, Ethyl, Propyl, Butyl, Pentyl, Hexyl;
R⁴, R⁵: Methyl, Ethyl, Propyl, Butyl, Pentyl, Hexyl, Cyclohexyl;
Y⁻ steht für R⁶COO⁻;
R⁶: tert. Butyl, Pentyl, iso-Pentyl, Hexyl, iso-Hexyl, Ethylpentyl, iso-Heptyl.

2. Verfahren gemäß Anspruch 1,
**dadurch gekennzeichnet,**
**dass** cycloaliphatische Diisocyanate eingesetzt werden, die nach dem Phosgenverfahren oder einem phosgenfreien Prozeß hergestellt wurden.

3. Verfahren gemäß Anspruch 1,
**dadurch gekennzeichnet,**
**dass** aliphatische Diisocyanate eingesetzt werden, die nach dem Phosgenverfahren oder einem phosgenfreien Prozeß hergestellt wurden.

4. Verfahren gemäß Anspruch 1,
**dadurch gekennzeichnet,**
**dass** Mischungen von aliphatischen und cycloaliphatischen Diisocyanaten eingesetzt werden, die nach dem Phosgenverfahren oder einem phosgenfreien Prozeß hergestellt wurden.

5. Verfahren nach mindestens einem der Ansprüche 1 bis 4,
**dadurch gekennzeichnet,**
**dass** als Diisocyanate 1-Isocyanato-3,3,5-trimethyl-5-isocyanatomethylcyclohexan (IPDI), 1,6-Hexamethylendiisocyanat (HDI), 1,5-Diisocyanato-2-methylpentan (MPDI), 2,5(2,6)-Bis(isocyanatomethyl)bicyclo[2.2.1]heptan (NBDI), 2,2,4- und/oder 2,4,4-Trimethyl-1,6-hexamethylendiisocyanat (TMDI) eingesetzt werden.

6. Verwendung der gemäß Anspruch 1-5 erhältlichen Verfahrensprodukte, gegebenenfalls in mit Blockierungsmitteln für Isocyanatgruppen blockierter Form, als Isocyanatkomponente in Polyurethanlacken und Polyurethanbeschichtungen.

7. Verbindungen, verwendbar als Katalysator zur Trimerisierung von Diisocyanaten der allgemeinen Formel (**I**) 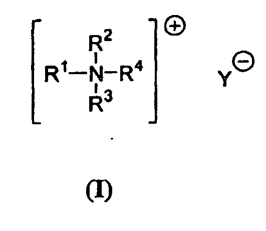
mit
R¹= 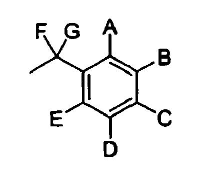
und wobei die Variablen folgende Bedeutung haben:
A: Methyl, Methoxy, H;
B: H;
C: Methyl, Methoxy, H;
D: H,
E: Methyl, Methoxy, H;
F: H, Methyl;
G: H, Methyl;
R², R³: R¹ oder Methyl, Ethyl, Propyl, Butyl, Pentyl, Hexyl;
R⁴, R⁵: Methyl, Ethyl, Propyl, Butyl, Pentyl, Hexyl, Cyclohexyl;
Y⁻ steht für R⁶COO⁻ ;
R⁶: tert. Butyl, Pentyl, iso-Pentyl, Hexyl, iso-Hexyl, Ethylpentyl, iso-Heptyl.

8. Verbindungen, verwendbar als Katalysator nach Anspruch 7,
**dadurch gekennzeichnet,**
**dass** die folgenden Individuen erfasst sind:
Benzyldimethylethylammoniumpivalat,
Benzyldimethylethylammonium-2-ethylhexanoat,
Benzyltributylammonium-2-ethylhexanoat,
N,N-Dimethyl-N-ethyl-N-(4-methoxybenzyl)ammonium-2-ethylhexanoat,
N-N-N-Tributyl-N-(4-methoxybenzyl)ammoniumpivalat

9. Verfahren zur Herstellung der Katalysatoren nach mindestens einem der Ansprüche 7 bis 8,
**dadurch gekennzeichnet,**
**dass** in einem ersten Schritt ein tertiäres Amin quaternisiert wird und in einem zweiten Schritt die erhaltene quaternäre Benzylammoniumverbindung in das entsprechende Carboxylat überführt wird.

## Claims

1. A process for preparing a low-viscosity and reduced-colour polyisocyanate containing isocyanurate groups by partially trimerizing an aliphatic and/or cycloaliphatic diisocyanate and then separating off excess diisocyanate, **characterized in that** the trimerization is performed in the presence of from 0.02 to 2% by weight, based on the weight of the diisocyanate used, of at least one trimerization catalyst of the general formula **(I)** 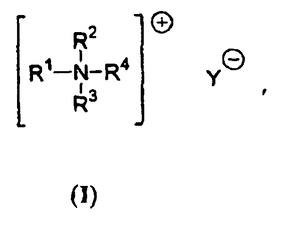
R¹ being 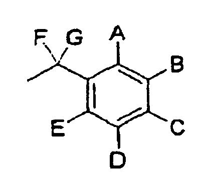
and the variables being defined as follows:
A: methyl, methoxy, H;
B: H;
C: methyl, methoxy, H;
D: H;
E methyl, methoxy, H;
F: H, methyl;
G: H, methyl;
R², R³: R¹ or methyl, ethyl, propyl, butyl, pentyl, hexyl;
R⁴, R⁵: methyl, ethyl, propyl, butyl, pentyl, hexyl, cyclohexyl;
Y⁻ is R⁶COO-;
R⁶:tert-butyl, pentyl, isopentyl, hexyl, isohexyl, ethylpentyl, isoheptyl.

2. A process according to claim 1, **characterized in that** a cycloaliphatic diisocyanate is used which has been prepared by the phosgene process or by a phosgene-free process.

3. A process according to claim 1, **characterized in that** an aliphatic diisocyanate is used which has been prepared by the phosgene process or by a phosgene-free process.

4. A process according to claim 1, **characterized in that** a mixture of aliphatic and cycloaliphatic diisocyanates is used which has been prepared by the phosgene process or by a phosgene-free process.

5. A process according to at least one of claims 1 to 4, **characterized in that** 1-isocyanato-3,3,5-trimethyl-5-isocyanatomethylcyclohexane (IPDI), 1,6-hexamethylene diisocyanate (HDI), 2-methylpentane 1,5-diisocyanate (MPDI), 2,5(2,6)-bis(isocyanatomethyl)bicyclo[2.2.1]heptane (NBDI), 2,2,4- and/or 2,4,4-trimethyl-1,6-hexamethylene diisocyanate (TMDI) are used as diisocyanate.

6. The use of the process product obtainable according to claim 1-5, in a form if desired in which it has been blocked with a blocking agent for isocyanate groups, as an isocyanate component in a polyurethane coating material or polyurethane coating.

7. A compound which can be used as catalyst for trimerizing a diisocyanate, of the general formula (I) 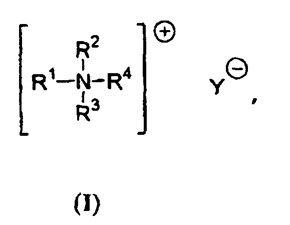
R¹ being 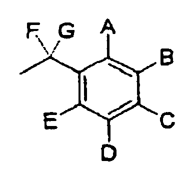
and the variables being defined as follows:
A: methyl, methoxy, H;
B: H;
C: methyl, methoxy, H;
D: H;
E methyl, methoxy, H;
F: H, methyl;
G: H, methyl;
R², R³: R¹ or methyl, ethyl, propyl, butyl, pentyl, hexyl;
R⁴, R⁵: methyl, ethyl, propyl, butyl, pentyl, hexyl, cyclohexyl;
Y⁻ is R⁶COO-;
R⁶:tert-butyl, pentyl, isopentyl, hexyl, isohexyl, ethylpentyl, isoheptyl.

8. A compound which can be used as catalyst according to claim 7, **characterized in that** the following individual compounds are embraced:
benzyldimethylethylammonium pivalate,
benzyldimethylethylammonium 2-ethylhexanoate,
benzyltributylammonium 2-ethylhexanoate,
N,N-dimethyl-N-ethyl-N-(4-methoxybenzyl)ammonium 2-ethylhexanoate,
N,N,N-tributyl-N-(4-methoxybenzyl)ammonium pivalate.

9. A process for preparing the catalyst according to at least one of claims 7 to 8, **characterized in that** in a first step a tertiary amine is quaternized and in a second step the resulting quaternary benzylammonium compound is converted into the corresponding carboxylate.

## Revendications

1. Procédé de fabrication de polyisocyanates contenant des groupes isocyanaturates, faiblement visqueux et à couleur réduite, par trimérisation partielle de diisocyanates aliphatiques et/ou cycloaliphatiques et séparation subséquente du diisocyanate excédentaire,
**caractérisé en ce que**
la trimérisation est réalisée en présence de 0,02 à 2 % en poids, sur la base du poids du diisocyanates mis en oeuvre, d'au moins un catalyseur de trimérisation de formule générale (I) 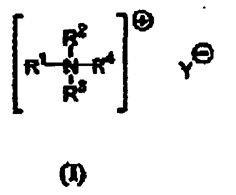
avec
R¹= 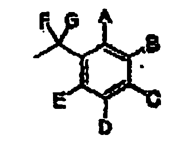
et les variables ayant la signification suivante :
A : méthyle, méthoxy, H ;
B : H ;
C : méthyle, méthoxy, H ;
D : H ;
E : méthyle, méthoxy, H ;
F : H, méthyle ;
G : H, méthyle ;
R², R³ : R¹ ou méthyle, éthyle, propyle, butyle, pentyle, hexyle ;
R⁴, R⁵ : méthyle, éthyle, propyle, butyle, pentyle, hexyle, cyclohexyle ;
Y représente un groupe R⁶COO ;
R⁶ : tert-butyle, pentyle, iso-pentyle, hexyle, iso-hexyle, éthylpentyle, iso-heptyle.

2. Procédé selon la revendication 1,
**caractérisé en ce qu'**
on utilise des diisocyanates cycloaliphatiques qui ont été fabriqués d'après le procédé phosgène ou un processus sans phosgène.

3. Procédé selon la revendication 1,
**caractérisé en ce qu'**
on utilise des diisocyanates aliphatiques qui ont été fabriqués d'après le procédé phosgène ou un processus sans phosgène.

4. Procédé selon la revendication 1,
**caractérisé en ce qu'**
on utilise des mélanges de diisocyanates aliphatiques et cycloaliphatiques qui ont été fabriqués d'après le procédé phosgène ou un processus sans phosgène.

5. Procédé selon au moins l'une quelconque des revendications 1 à 4,
**caractérisé en ce que**
comme diisocyanates, on utilise du 1-isocyanato-3,3,5-triméthyl-5-isocyanatométhylcyclohexane (IPDI), du diisocyanate de 1,6-hexaméthylène (HDI), du 1,5-diisocyanato- 2-méthylpenthane (MPDI), du 2,5(2,6)-bis(isocyanatométhyl)bicyclo-[2,2,1]heptane (NBDI), du diisocyanate de 2,2,4 et/ou 2,4,4-triméthyl-1,6-hexaméthylène (TMDI).

6. Utilisation des produits du procédé pouvant être obtenus suivant la revendication 1-5, le cas échéant sous forme bloquée avec des agents de blocage pour groupes isocyanates, en tant que composant isocyanate dans des laques à base de polyuréthane et des revêtements à base de polyuréthane.

7. Composés, utilisables en tant que catalyseur pour la trimérisation de diisocyanates de formule générale (I) 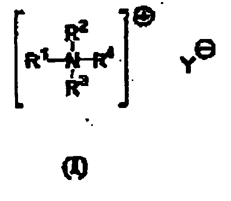
avec
R¹= 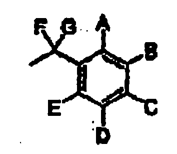
et les variables ayant a signification suivante :
A : méthyle, méthoxy, H ;
B:H;
C : méthyle, méthoxy, H ;
D : H ;
E : méthyle, méthoxy, H ;
F : H, méthyle ;
G : H, méthyle ;
R², R³ : R¹ ou méthyle, éthyle, propyle, butyle, pentyle, hexyle ;
R⁴, R⁵ : méthyle, éthyle, propyle, butyle, pentyle, hexyle, cyclohexyle ;
Y représente un groupe R⁶COO ;
R⁶ : tert-butyle, pentyle, iso-pentyle, hexyle, iso-hexyle, éthylpentyle, iso-heptyle.

8. Composés utilisables en tant que catalyseur selon la revendication 7,
**caractérisés en ce que**
les individus suivants sont détectés :
pivalate de benzyldiméthyléthylammonium,
2-éthylhexanoate de benzyldiméthyléthylammonium,
2-éthyl-hexanoate de benzyltributylammonium,
2-éthylhexanoate de N,N-diméthyl-N-éthyl-N-(4-méthoxybenzyl)ammonium,
pivalate de N-N-N-tributyl-N-(4-méthoxybenzyl)ammonium.

9. Procédé de fabrication des catalyseurs selon au moins l'une quelconque des revendications 7 à 8,
**caractérisé en ce que**
dans une première étape, on quaternise une amine tertiaire et, dans une deuxième étape, on transforme le composé benzylammonium quaternaire obtenu en carboxylate correspondant.
